# EUROPÄISCHE PATENTANMELDUNG

(11) **EP 4 702 941 A1**
(43) Veröffentlichungstag der Anmeldung: **04.03.2026**
(21) Anmeldenummer: 24196861.9
(22) Anmeldetag: 28.08.2024
(51) Int. Cl.: A61B 18/12, H02M 5/12

(54) **GENERATOR MIT KENNLINIENUMSCHALTUNG**

(71) Anmelder: Erbe Elektromedizin GmbH, 72072 Tübingen (DE)
(72) Erfinder: DORNHOF, Konstantin, 72160 Horb am Neckar (DE); SELIG, Peter, 72147 Nehren (DE)
(74) Vertreter: Rüger Abel Patentanwälte PartGmbB

(57) **Zusammenfassung**

Mit dem erfindungsgemäßen Konzept wird ein Generator (11) zur Speisung verschiedener Instrumente (35, 44) vorgeschlagen, der ohne Zuhilfenahme einer Regelschleife gewünschte Ausgangskennlinien bereitstellt. Dazu nutzt er ein reaktives Netzwerk (19), dass die gewünschten Kennlinien von sich aus (d.h. intrinsisch) hat. Dies wird erreicht, indem in dem Ausgangszweig des Generators (11) verschieden wahlweise einschaltbare komplexe Widerstände vorgesehen sind, die unterschiedliche Zusammenhänge zwischen abgegebener Leistung und Lastwiederstand bereitstellen.

## Beschreibung

Die Erfindung betrifft einen Generator zur Speisung eines elektrochirurgischen Instruments mit Strom, zur Bewirkung einer Gewebeveränderung, insbesondere einer devitalisierenden Gewebeveränderung. Außerdem betrifft die Erfindung ein System, bestehend aus dem genannten Generator und mehreren an den Generator anschließbaren Instrumenten, die unterschiedlichen Aufgaben erfüllen und dazu seitens des Generators unterschiedliche Strom/Zeit-Verläufe, Spannungs/Zeit-Verläufe, Strom/Spannungs-Kennlinien oder unterschiedliche Zusammenhänge von Gewebewiderstand und ins Gewebe eingebrachter Leistung (Gewebewiderstands/Leistungs-Kennlinien) benötigen.

Elektrochirurgische Instrumente, wie beispielsweise Elektroskalpelle, Kauterisierungszangen oder dergleichen sind bekannt. Zur Speisung solcher Instrumente sind verschiedene Generatorkonzepte bekannt.

Die EP 2 499 982 A1 offenbart dazu einen Generator mit einer Sensorschaltung, die mehrere Sensoren enthält, um Gewebe- und Energieeigenschaften zu erfassen, wie beispielsweise die Gewebeimpedanz, die Gewebetemperatur, den abgegebenen Strom und/oder die abgegebene Spannung. Diese Sensorschaltung liefert ein Rückführungssignal an die Generatorsteuerung. Durch diese Rückführung ist eine Regelschleife gebildet, die die Stromabgabe des das Instrument speisenden Generators wunschgemäß regelt.

Ein ähnlicher Generator ist aus der EP 1 862 137 A1 bekannt. Auch dieser Generator nutzt eine Sensorschaltung, die die Spannung und den Strom am Generatorausgang erfasst und den Generator dann entsprechend steuert. So auch der Generator nach der EP 1 051 948 A2.

Die EP 2 520 241 B1 sieht ebenfalls eine Regelschleife zur Regelung des Betriebs des Generators vor, wobei die Regelschleife dazu dient, einen gewünschten Zusammenhang zwischen dem durch das Gewebe fließenden Strom und der anliegenden Spannung herzustellen, wobei diese Kennlinien linear oder nichtlinear festlegbar sind.

Weiter offenbart die EP 2 405 842 B1 einen Generator mit ausgangsseitigem Transformator, dem zur Anpassung an eine anzuschließende Last ein Reihenschwingkreis nachgeschaltet ist. In einer der dargestellten Ausführungsformen kann der Schwingkreis über Schalter mit verschiedenen Anzapfungen des ausgangsseitigen Transformators des Generators verbunden werden.

Generatoren der genannten Art sollen häufig in der Lage sein, verschiedenartige Instrumente mit chirurgischem Strom zu versorgen. Dabei benötigen beispielsweise Kauterisierungsinstrumente grundsätzlich andere Werte und zeitliche Verläufe von Spannung und Strom als beispielsweise Elektroskalpelle, die wiederum andere Verläufe von Spannung und Strom benötigen als beispielsweise Ablationsinstrumente oder Plasmasonden. Deswegen weisen derartige Generatoren typischerweise einen Mode-Wahlschalter zur Auswahl verschiedener Modes (Koagulieren, Schneiden, Kauterisieren usw.) auf, mit dem der Generatorsteuerung verschiedene Werte und Zeitverläufe für Strom, Spannung oder sonstige elektrische Parameter (Frequenz, Modulation, Crestfaktor, Leistung oder Grenzwert für eine oder mehrerer dieser Parameter) vorgegeben werden.

Zur Realisierung eines gewünschten ausgangsseitigen Verhaltens der Generatoren dienen Regelschleifen, die aber bauartbedingten Restriktionen unterliegen. Beispielsweise können bei schnellen Laständerungen Regelschwingungen auftreten, die zu zeitweilig erheblichen Abweichungen zwischen dem gewünschten Strom und dem tatsächlich fließenden Strom führen. Wenn aber die Spannung oder der Strom wenn auch nur kurzzeilig, z.B. im Rahmen einer Regelschwingung stark vom Sollwert abweichen, können unerwünschte Behandlungseffekte auftreten. Beispielsweise können beim Koagulieren Klebeeffekte auftreten. Klebt eine Elektrode am Gewebe kann das Losreißen zu unerwünschten Läsionen führen, die das Operationsergebnis beeinträchtigen können. Auch beim Schneiden können durch zu starke oder stellenweise zu schwache Schnittrandkoagulation Blutungen oder auch Klebeeffekte auftreten, die unerwünscht sind.

Davon ausgehend ist es Aufgabe der Erfindung, einen verbesserten Generator anzugeben.

Diese Aufgabe wird mit dem Generator nach Anspruch 1 gelöst:
Der erfindungsgemäße Generator weist ausgangsseitig ein reaktives Netzwerk auf, das mehrere Induktoren und mehrere Kondensatoren umfasst, die in wahlweiser Paarung mit dem Generatorausgang verbunden werden können. Jede Paarung wird durch die Reihenschaltung wenigstens eines der Induktoren und eines aus einer Gruppe von Kondensatoren ausgewählten Kondensators gebildet. Das Ausgangsnetzwerk des Generators legt, je nachdem welcher Schaltungszweig des Ausgangsnetzwerks aktiviert ist, unterschiedliche Innenwiderstände des Generators fest, die zu unterschiedlichen Ausgangskennlinien führen. Dies ohne Rückführung von Messwerten von Strom und Spannung auf den Taktgeber oder den gesteuerten Schalter zur Erregung des Schwingkreises. Der für den Betrieb eines spezifischen Instruments erforderliche Zusammenhang zwischen dem Gewebewiderstand und der in das Gewebe eingebrachten Leistung wird dann ausschließlich von dem sich im Behandlungsverlauf zeitlich ändernden Gewebewiderstand herbeigeführt. Mit anderen Worten, die innere Impedanz, d.h. der komplexe Innenwiderstand des Generators und somit die Ausgangskennlinie sind modeentsprechend so festgelegt, dass an dem, angeschlossenen Instrument der gewünschte chirurgische Effekt ohne Regeleingriff erreicht wird.

Dies führt dazu, dass einerseits die verschiedenen Modes (Schneiden, Koagulieren, usw.) schnell und einfach auswählbar sind und andererseits Laständerungen bzw. Lastströme während der Anwendung nicht zu Regelschwingungen und eventuell unerwünschten chirurgischen Effekten führen. Der vom Generator abgegebene Behandlungsstrom resultiert aus dem direkten Zusammenspiel von Generator-Innenwiderstand und Gewebewiderstand. Beispielsweise können in einer Generatoreinstellung Koagulations- und Dissektionsinstrumente, wie sie beispielsweise zur Gefäßversiegelung und Gefäßtrennung, verwendet werden, über den erfindungsgemäßen Generator mit Strom und Spannung versorgt werden, ohne dass zur Festlegung der Spannung oder des Stroms ein Regeleingriff nötig wäre. Der sich ändernde Widerstand des behandelten Gewebes führt zu einer Änderung der an den Elektroden anliegenden Spannung während des Behandlungsverlaufs, wobei die Spannungsänderungen durch entsprechende Kennliniengestaltung dem Behandlungsverlauf entsprechen, die dem chirurgischen Prozess zuträglich sind. In einer anderen Generatoreinstellung kann ein anderes Instrument, z.B. ein Elektroskalpell, ebenfalls ohne Regeleingriff gespeist werden.

Der primärseitige Induktor wird nachfolgend als Primärinduktor bezeichnet. Der primärseitige Kondensator wird nachfolgend als Primärkondensator bezeichnet. Der Primärinduktor und der Primärkondensator bilden einen Parallelschwingkreis. Dieser Parallelschwingkreis ist mit einem oder mehreren elektronischen Schalter(n) verbunden, die dazu genutzt werden, den Parallelschwingkreis zur Schwingung anzuregen. Der (mindestens eine) elektronische Schalter wird von einem Taktgeber alternierend geöffnet und geschlossen, wobei das dazu von dem Taktgeber abgegebene Schaltsignal vorzugsweise eine vorgegebene Frequenz aufweist. Die vorgegeben Frequenz ist vorzugsweise zeitlich unveränderlich und somit eine Festfrequenz. Es ist jedoch möglich, den Taktgeber so auszugestalten, dass das Taktsignal einer Modulation unterliegt, beispielsweise einer Pulsbreitenmodulation. Außerdem kann es mit einer anderen Frequenz amplitudenmoduliert sein, beispielsweise ein/aus-getastet, wobei auch diese Modulationsfrequenz pulsbreitenmoduliert sein kann. Die Modulation der Taktgebers kann entsprechend der gewählten Betriebsart (Mode) eingestellt sein. Wiederum können die Modulationen der verschiedenen Modes fest vorgegeben sein.

Die sekundärseitigen Induktoren des Generators werden nachfolgend als Sekundärinduktoren bezeichnet. Die Sekundärinduktoren sind eng an den Primärinduktor angekoppelt. Der Koppelfaktor liegt dabei vorzugsweise bei über 0,9, vorzugsweise über 0,95 und am besten bei oder über 0,97. Die Windungszahlen der Induktoren richten sich nach der im Leerlauf des Generators gewünschten Leerlaufspannung. Die Windungszahl eines Primärinduktors ist vorzugsweise kleiner als die Windungszahl des Primärinduktors. Zumindest vorzugsweise ist auch die Summe der Windungszahlen aller Sekundärinduktoren höchstens so groß wie die Anzahl der Windungen des Primärinduktors.

Die sekundärseitigen Kondensatoren werden hier als Sekundärkondensatoren bezeichnet. Jeweils ein oder mehrere Sekundärinduktoren sind über einen Schaltungszweig mit einem Ausgangspol des Generators verbunden. Der Schaltungszweig enthält eine Schaltstrecke eines Wahlschalters und einen Sekundärkondensator. Diese Reihenschaltungen können gleiche oder unterschiedliche Resonanzfrequenzen aufweisen. Durch die starke Kopplung der Primär- und Sekundärinduktoren werden die Sekundärkondensatoren in den Primärkreis transformiert und verringern damit dessen Resonanzfrequenz. Vorzugsweise liegt die Schaltfrequenz des wenigstens einen zur Anregung des Primärschwingkreises dienenden Schalters über der Resonanzfrequenz der aus dem Primärschwingkreis und den Sekundärkondensatoren gebildeten schwingfähigen Einheit. Dies gilt für zumindest einen oder mehrere Modes, vorzugsweise für alle.

Bei dem erfindungsgemäßen Generator kann sich die Resonanzfrequenz der oben genannten schwingfähigen Einheit in Abhängigkeit von der Impedanz des bestromten Gewebes ändern. Dieser Effekt kann dazu genutzt werden, den gewünschten Zusammenhang zwischen dem Gewebewiderstand und der in das Gewebe eingetragenen Leistung herzustellen.

Durch Aktivierung einer der genannten Reihenschaltungen (d.h. Einschalten der jeweiligen Schaltstrecke) und Deaktivierung der anderen Reihenschaltungen (Ausschalten der jeweiligen Schaltstrecke) wird die Ausgangskennlinie des Generators maßgeblich beeinflusst. Dabei ist es möglich, den verschiedenen Reihenschaltungen Kennlinien zu verleihen, die unterschiedliche Behandlungsmodes ermöglichen. Beispielsweise kann der Generator somit sowohl für Koagulationsinstrumente als auch für Dissektionsinstrumente sowie weitere Instrumente genutzt werden, ohne die geforderte Ausgangskennlinie über eine Regelschleife erzeugen zu müssen. Vielmehr wird die jeweilige Ausgangskennlinie allein von dem reaktiven Netzwerk bereitgestellt, das aus dem primärseitigen Schwingkreis sowie der ausgangsseitig jeweils aktivierten Reihenschaltung besteht.

Zusätzlich kann der Modewahlschalter mit dem Taktgeber verbunden sein. Alternativ kann ein den Modewahlschalter steuerndes Signal dem Taktgeber zugeleitet sein. In beiden Fällen kann der Taktgeber darauf eingerichtet sein, ein entsprechend den Erfordernissen des gewählten Modes angepasstes Taktsignal zu liefern.

Weitere Einzelheiten von vorteilhaften Ausführungsformen der Erfindung sind Gegenstand der Zeichnung, der zugehörigen Beschreibung oder von Ansprüchen. In der Zeichnung zeigen:
Figur 1 ein Übersichtsschaubild des erfindungsgemäßen Generators mit angeschlossenem Instrument, in symbolischer Darstellung,
Figur 2 den Generator nach Figur 1, in einer weiteren symbolischen Darstellung,
Figur 3 den Generator nach Figur 1 und 2 als Übersichtsschaltplan, angeschlossen an ein erstes Instrument,
Figur 4 den Generator nach Figur 3 angeschlossen an ein anderes Instrument,
Figur 5 verschiedene Ausgangskennlinien des Generators in den Einstellungen nach Figur 3 und 4,
Figur 6 ein Ersatzschaltbild zur Veranschaulichung der Funktion des Generators nach Figur 3 und 4,
Figur 7 Resonanzfrequenzen des Generatorschwingkreises bei verschiedenen Einstellungen,
Figur 8 Schaltsignalfolgen des Taktgebers des Generators.

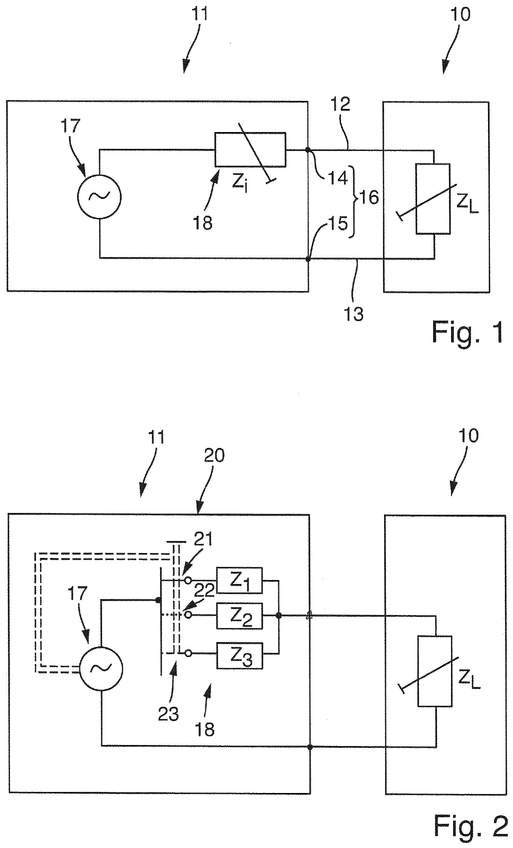

In Figur 1 sind ein aus einem Patienten und einem Instrument gebildeter Lastwiderstand 10 und ein den Lastwiderstand 10 speisender Generator 11 symbolisch veranschaulicht. Der Lastwiderstand 10 weist eine Lastimpedanz Z_{L} auf, die sowohl vom Gewebetyp als auch von der Behandlungsart als auch vom Behandlungsfortschritt, d.h. der verstrichenen Zeit und der Intensität der Bestromung abhängig ist. Die Behandlungsart hat insoweit Einfluss auf den Gewebewiderstand Z_{L}, als die Form und Größe der Elektrode, die Intensität des Kontakts zwischen der Elektrode und dem Gewebe und der Zustand des Gewebes (nass, trocken, koaguliert usw.) eine Rolle spielen. Die Lastimpedanz ist deswegen in Figur 1 als veränderlicher komplexer Widerstand dargestellt.

Der Lastwiderstand 10 ist über zwei Leitungen 12, 13 an zwei Pole 14, 15 eines Generatorausgangs 16 angeschlossen. Der Generator 11 enthält eine hochfrequente Spannungsquelle 17, deren komplexer Innenwiderstand 18 in Figur 1 als gesondertes Schaltsymbol veranschaulicht ist. Der komplexe Innenwiderstand 18 weist eine Impedanz Zᵢ auf, die eine lineare oder auch nichtlineare Strom/Spannungs-Kennlinie haben kann.

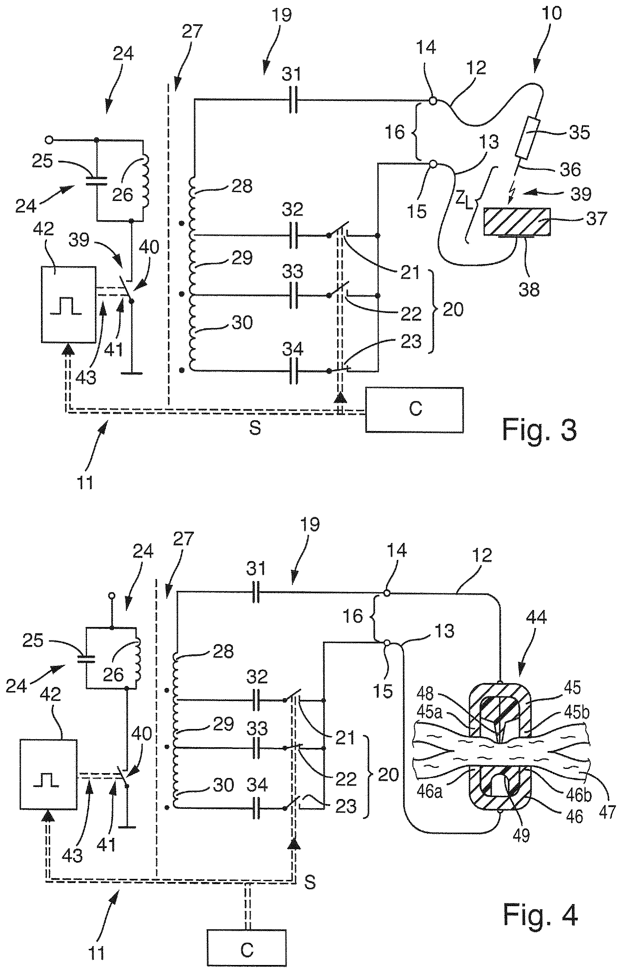

Der Innenwiderstand 18 ist in Stufen veränderbar, sodass er, wie Figur 2 andeutet, diskrete unterschiedliche Impedanzen Z₁, Z₂, Z₃ annehmen kann. Die Impedanzen Z₁, Z₂, Z₃ des Innenwiderstands 18 können durch verschiedene Zweige eines reaktiven Netzwerks 19 gebildet sein, das in Figur 3 veranschaulicht ist. Zur Aktivierung oder Deaktivierung der verschiedenen Zweige dient ein Wahlschalter 20, der verschiedene Schaltstrecken 21, 22, 23 aufweist. Der Wahlschalter 20 kann ein manueller Schalter oder ein von einem Steuersignal S gesteuerter Schalter sein. Zur Erzeugung des Steuersignals kann eine Steuerbaugruppe C dienen.

Die Anzahl der Schaltstrecken 21 bis 23 richtet sich nach der Anzahl der verschiedenen zu realisierenden Impedanzen Z₁ bis Z₃ des Innenwiderstands 18 und somit der Anzahl der zu realisierenden Ausgangskennlinien und entsprechenden Modes des Generators 11. Der Wahlschalter 20 ist dabei so beschaffen, dass von seinen Schaltstrecken 21 bis 23 immer lediglich eine elektrisch leitend (durchlässig) sein kann, während alle anderen Schaltstrecken undurchlässig (gesperrt) sind. Bei den Schaltern kann es sich um kontaktlose elektronische Schalter, um mechanische Schalter mit Schaltkontakt oder auch um eine Buchsen-Anordnung handeln, die für eine der Leitungen 12, 13 mehrere Pole zur Auswahl bietet. Zur Steuerung des Wahlschalters 20 kann ein Steuersignal S dienen, das von einem manuell betätigbaren Schalter oder einer nicht weiter veranschaulichten Generatorsteuerung bereitgestellt ist.

Zu dem Generator 11 gehört ein primärseitiger Schwingkreis 24, zu dem ein Primärkondensator 25 und ein Primärinduktor 26 gehören, die elektrisch zueinander parallelgeschaltet sind. Der Schwingkreis 24 ist auf eine Resonanzfrequenz von mehreren 100 kHz abgestimmt, z.B. auf eine Frequenz von 480 kHz. Dazu kann der Primärkondensator 25 einen Wert von 2,2 nF und der Primärinduktor 26 einen Wert von 50 µH haben. Andere Werte für die Resonanzfrequenz, den Primärkondensator 25 und den Primärinduktor 26 sind aber möglich.

Der Primärinduktor 26 wird vorzugsweise durch die Primärwicklung eines Hochfrequenztransformators 27 gebildet. Der Hochfrequenztransformator 27 weist mehrere Sekundärwicklungen auf, die Sekundärinduktoren 28, 29, 30 bilden und induktiv mit dem Primärinduktor 26 koppeln. Die Sekundärinduktoren 28, 29, 30 können gleiche oder unterschiedliche Windungszahlen und somit gleiche oder unterschiedliche Induktivitätswerte aufweisen. Sie können als Einzelspulen gewickelt oder durch eine einzige Spule gebildet sein, die mehrere Anzapfungen aufweist und die Spule somit in die einzelnen Sekundärinduktoren 28, 29, 30 unterteilt. Die Anzahl der Sekundärinduktoren 28, 29, 30 stimmt mit der Anzahl der Schaltstrecken 21 bis 23 überein und kann entsprechend der Anzahl der gewünschten zu realisierenden Ausgangskennlinien des Generators 11 variieren. Die Sekundärinduktoren 28, 29, 30 haben vorzugsweise jeweils eine Windungszahl, die geringer ist, als die Windungszahl des Primärinduktors 26. Weiter vorzugsweise ist die Summe der Windungszahlen der Sekundärinduktoren 28, 29, 30 nicht wesentlich größer, im bevorzugten Falls sogar höchstens so groß, wie die Windungszahl des Primärinduktors 26.

Der erste Induktor 28 ist über einen Koppelkondensator 31 mit dem Pol 14 des Generatorausgangs 16 verbunden. Das andere Ende des Induktors 28 ist über einen Sekundärkondensator 32 und die Schaltstrecke 21 des Wahlschalters 20 mit dem anderen Pol 15 des Generatorausgangs 16 verbunden. Somit bilden der Sekundärinduktor 28 und der Sekundärkondensator 21 eine erste induktiv gespeiste Reihenschaltung zur wahlweisen Speisung des Ausgangs 16. Die Reihenfolge des Sekundärkondensators 32 und der Schaltstrecke 21 kann wie in Figur 3 dargestellt oder auch umgekehrt sein.

Die Wicklungsenden der Sekundärinduktoren 28, 29, 30 sind in Figur 3 jeweils durch einen Punkt markiert. Dies hat Bedeutung für die nachstehende Erläuterung der Zusammenschaltung der Sekundärinduktoren 28 bis 30.

Der Wicklungsanfang des Sekundärinduktors 29 ist mit dem Wicklungsende des Sekundärinduktors 28 verbunden. Ebenso ist der Wicklungsanfang des Sekundärinduktors 30 mit dem Wicklungsende des Sekundärinduktors 29 verbunden. Von dem Wicklungsende des Sekundärinduktors 29 geht ein Schaltungszweig ab, in dem ein weiterer Sekundärkondensator 33 angeordnet ist, der mit dem Sekundärinduktor 29 und dem Sekundärinduktor 28 eine Reihenschaltung bildet. Über die Schaltstrecke 22 kann diese Reihenschaltung mit dem Pol 15 des Generatorausgangs 16 verbunden werden. Die Reihenfolge des Sekundärkondensators 33 und der Schaltstrecke 22 kann wie in Figur 3 dargestellt oder auch umgekehrt sein.

Das Wicklungsende des Sekundärinduktors 30 bildet mit einem dritten Sekundärkondensator 34 eine Reihenschaltung, die über die Schaltstrecke 23 des Wahlschalters 20 wahlweise mit dem Pol 15 des Generatorausgangs 16 verbunden werden kann. Die Reihenfolge des Sekundärkondensators 34 und der Schaltstrecke 23 kann wie in Figur 3 dargestellt oder auch umgekehrt sein. Es können in ähnlicher Weise weitere Sekundärinduktoren, Sekundärkondensatoren und Schaltstrecken vorgesehen sein.

Die Sekundärinduktoren 28, 29, 30 können gleiche oder unterschiedliche Werte aufweisen und koppeln induktiv mit dem Primärinduktor 26. Der Koppelfaktor ist vorzugsweise größer als 0.95, weiter vorzugsweise größer als 0.97. Die Sekundärkondensatoren 32, 33, 34 weisen absteigend gestaffelte Werte auf. Der Sekundärkondensator 32 ist größer als der Sekundärkondensator 33, welcher wiederum größer ist als der Sekundärkondensator 34. Der Koppelkondensator 31 ist größer als alle Sekundärkondensatoren. Insbesondere kann er größer sein als die Summe der Kapazitäten aller Sekundärkondensatoren 32 bis 34. Dies gilt auch, wenn anders als dargestellt, nicht nur drei verschiedene Schaltungszweige mit drei Schaltstrecken 21 bis 23 und somit auch mehrere Sekundärinduktoren und mehrere Sekundärkondensatoren vorhanden sind. Die Windungszahlen der Sekundärinduktoren 28, 29, 30 sind so auf die Windungszahl des Primärinduktors 26 abgestimmt, dass die Spannung an den Sekundärinduktoren 28 - 30 höchstens so groß ist, wie die Spannung in dem primären (Parallel-)Schwingkreis 24. Die Induktoren 26, 28, 29, 30 bilden somit einen Transformator, der die Schwingkreisspannung mit Faktoren von z.B. 1,5:1, 2:1, 3:1 oder anderen Verhältnissen heruntertransformiert oder höchstens 1:1 auskoppelt. Damit wird umgekehrt die Gewebeimpedanz (zumindest wenn nicht der größte Übertragungsfaktor 1:1 zur Anwendung kommt) vergrößert in den Schwingkreis 24 transformiert und somit die Bedämpfung des Schwingkreises 24 durch die Gewebeimpedanz gemindert.

An dem Generatorausgang 16 ist ein chirurgisches Instrument 35 mit einer Elektrode 36 angeschlossen, das hier als monopolares Instrument ausgebildet ist und dazu dient, auf biologisches Gewebe 37 einzuwirken, beispielsweise einen Schnitt zu vollbringen. Das biologische Gewebe 37 (zum Beispiel in Gestalt eines lebenden Patienten) ist über eine Neutralelektrode 38 an den Generatorausgang 16 angeschlossen. Das Instrument 35 mit seiner Elektrode 36 sowie eine gegebenenfalls vorhandene Funkenstrecke 39 bilden zusammen mit dem biologischen Gewebe 37 und dem Übergangswiderstand zu der Neutralelektrode 38 den Lastwiderstand 10.

Zur Erregung des reaktiven Netzwerks 19, insbesondere zur Erregung des Schwingkreises 24, dient eine elektronische Schaltung, die mindestens einen elektronischen Schalter 39 aufweist. Dieser weist eine Steuerstrecke 40 und einen Steuereingang 41 auf, der an einen Taktgeber 42 angeschlossen ist, um von diesen ein Steuersignal 43 zu empfangen. Der Taktgeber 42 ist vorzugsweise dazu eingerichtet, das Schaltsignal 43 als Rechtecksignal mit einer festen Frequenz abzugeben. Diese Frequenz liegt vorzugsweise oberhalb 200 kHz und kann beispielsweise 350 kHz oder auch 480 kHz betragen. Vorzugsweise liegt die Taktfrequenz unter 5 MHz weiter vorzugsweise unter 1 MHz.

In der einfachsten Ausführungsform ist das von dem Taktgeber 42 erzeugte Schaltsignal 43 unveränderlich für alle gewählten Betriebsarten (Modes), d.h. unabhängig von der Schaltposition des Wahlschalters 20. Im bevorzugten Fall ist jedoch nicht nur eine Verbindung zwischen der Steuerbaugruppe C und dem Wahlschalter 20, sondern auch eine Verbindung zwischen der Steuerbaugruppe C und dem Taktgeber vorgesehen. Dadurch kann der Taktgeber 42 für jede gewählte Betriebsart (jeden Mode) ein geeignetes Taktsignal 43 abgeben. Die Taktsignale 43 der verschiedenen Modes können sich im der Modulation unterscheiden. Vorzugsweise handelt es sich um Rechteckwellen mit festgelegter Frequenz zwischen 100kHz und 5 MHZ, z.B. fest 350 kHz oder fest 480 kHz. Die Frequenz kann aber auch modeabhängig festgelegt sein. Vorzugsweise ist sie aber mindestens innerhalb eines Modes unveränderlich.

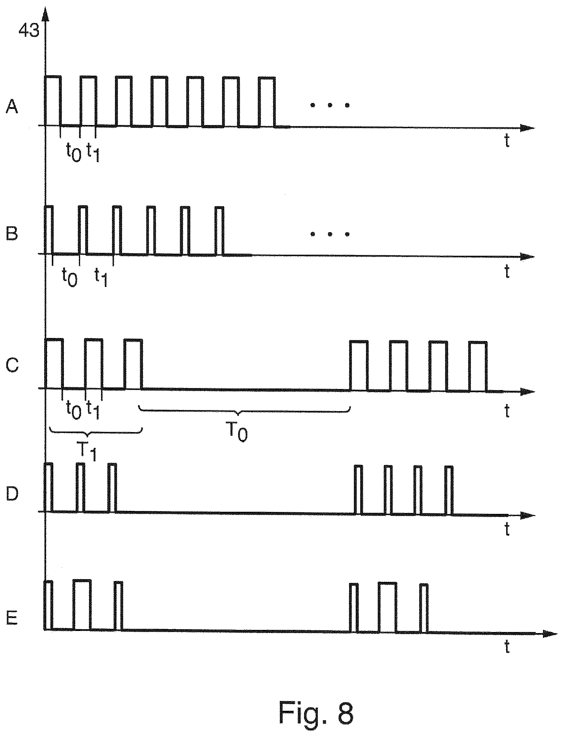

Die Taktsignale 43 der verschiedenen Modes können z.B. unmoduliert ("CW" - Rechteck Dauerstrich) mit unterschiedlichen Puls/Pause-Verhältnissen t₁/t₀ siehe Figur 8 sein. Das Taktsignal 43 kann nach den Mustern A oder B festgelegt sein. Die Taktsignale 43 können auch gruppenweise gepulst mit unterschiedlichen Gruppen-Puls/Pause-Verhältnissen T₁/T₀ sein, siehe Figur 8 Taktsignal 43 nach den Mustern C, D oder F.

Das Steuersignal 43 kann somit eine ununterbrochene Impulsfolge (A, B) oder auch eine gepulste Impulsfolge (C, D, F) sein. In diesem Fall ist das Taktsignal 43 ein/aus-getastet, das heißt mit einem Rechtecksignal multipliziert sein, dessen Frequenz geringer ist, als die Frequenz des Steuersignals 43. Diese Modulationsfrequenz kann pulsbreitenmoduliert sein, um Anpassungen an verschiedene Instrumentenanforderungen vorzunehmen. Außerdem kann das Steuersignal 43 ebenfalls pulsbreitenmoduliert sein, beispielsweise um Leistungsbegrenzungen oder Leistungsvorgaben zu erfüllen.

In Figur 3 ist der gleiche Generator 11 veranschaulicht, wobei an diesen Generator 11 ein anderes Instrument 44 angeschlossen ist, das beispielsweise als Fusions- und Dissektionsinstrument ausgebildet ist. Solche Instrumente sind als Bipolarinstrumente üblich, beispielsweise zum Versiegeln und Trennen von Gefäßen, zum Beispiel Blutgefäßen. Zu diesem Instrument 44 gehören zwei Branchen 45, 46, zwischen denen biologisches Gewebe 47, beispielsweise in Gestalt eines Blutgefäßes oder eines anderen Gefäßes, gefasst werden kann. Die Branchen 45, 46 weisen jeweils zwei in seitlichem Abstand zueinander angeordnete Teilelektroden 45a, 45b, 46a, 46b auf, die als Koagulations- und Fusionselektroden dienen und die dazu an die Leitungen 12, 13 angeschlossen sind. Zwischen den Teilelektroden 45a, 45b kann eine Schneidelektrode 48 angeordnet sein, die elektrisch mit der Branche 45 verbunden sein kann. Alternativ kann das Instrument eine Spannungsumsetzungseinrichtung, beispielsweise einen Transformator, enthalten, der aus den Leitungen 12, 13 gespeist wird und die Schneidelektrode 48 mit Strom versorgt.

Zwischen den Teilelektroden 46a, 46b kann ein vorzugsweise elastisch ausgebildetes Widerlager 49 angeordnet sein, welches das Gewebe 47 gegen die Schneidelektrode 48 drängt.

Der insoweit beschriebene Generator 11 arbeitet im Zusammenhang mit den verschiedenen Instrumenten 35, 44 wie folgt:
Bei Betrieb des Instruments 35 gibt die Steuerbaugruppe C ein Schaltsignal S an den Wahlschalter 20, so dass die Schaltstrecke 23 freigegeben ist, während die Schaltstrecken 21, 22 gesperrt sind. Es sind somit aus Sicht des Ausgangs 16 die Sekundärinduktoren 28, 29, 30 mit dem Sekundärkondensator 34 in Reihe geschaltet. Der Koppelkondensator 31 vervollständigt den Stromkreis. Primärseitig wird der elektronische Schalter 39 mit vorgegebener Taktfrequenz f alternierend geöffnet und geschlossen. Der Taktgeber 42 gibt dazu dasjenige Schaltsignal 43 aus, das für den gewählten Mode geeignet ist. Dies z.B. das Schaltsignal mit dem Muster A aus Figur 8.

Die Frequenz f des Schaltsignals 43 liegt dabei vorzugsweise in der Nähe der Resonanzfrequenz des Schwingkreises 24. Die Sekundärinduktoren 28, 29, 30 und der Sekundärkondensator 34 sowie die Lastimpedanz Z*_{L} transformieren sich mit dem Übersetzungsverhältnis des Transformators 27 in die Primärseite.

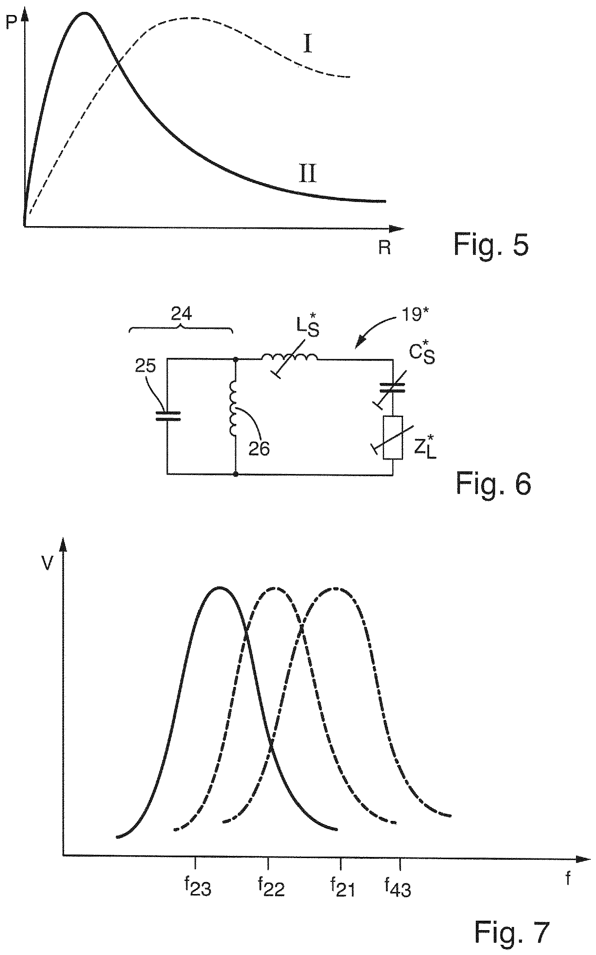

Das Ersatzschaltbild dazu ist in Figur 6 veranschaulicht. Es ergibt sich insgesamt ein reaktives transformiertes Netzwerk 19*, dessen Kennlinie I in Figur 5 veranschaulicht ist. Die Abszisse zeigt den Betrag R der Lastimpedanz Z*_{L} während die Ordinate P die an dem Gewebewiderstand umgesetzte elektrische Leistung (Scheinleistung) wiedergibt. Bei noch feuchtem Gewebe ist der Gewebewiderstand gering. Die am Gewebe umgesetzte Leistung ist deswegen noch gering, nimmt aber mit zunehmender Austrocknung des Gewebes stark zu, um bei mittleren Gewebewiderständen ihr Maximum zu erreichen. Damit können an der Elektrode 36 Funken unterhalten werden, die zum Gewebeschnitt führen.

Soll hingegen ein bipolares Koagulation- und Dissektionsinstrument an den Generatorausgang 16 angeschlossen werden, muss der entsprechende Mode gewählt werden. Dazu gibt die Steuerbaugruppe C ein Steuersignal S aus, infolge dessen der Wahlschalter 20 die Schaltstrecke 23 sperrt und dafür die Schaltstrecke 21 oder, wie in Figur 4 dargestellt, die Schaltstrecke 22 freigibt. Die niedrigere Induktivität der lediglich zwei Sekundärinduktoren 28, 29 in Verbindung mit der höheren Kapazität des Sekundärkondensators 33 führt nun zu einer veränderten Ausgangskennlinie II gemäß Figur 5. Außerdem kann das zugleich an den Taktgeber 42 geleitete Signal S dazu führen, dass dieser ein anderes Steuersignal 43, z.B. das Steuersignal nach dem Muster C in Figur 8 abgibt.

Die maximale ins Gewebe eingebrachte Leistung wird nun bereits bei niedrigeren Gewebewiderständen erreicht, was zu einer Koagulation des Gewebes zwischen den Teilelektroden 45a, 46a bzw. 45b, 46b führt. Die kleinflächige Schneidelektrode 48 übernimmt in diesem Zustand nur wenig Strom. Mit zunehmender Austrocknung des Gewebes nimmt die ins Gewebe eingebrachte Leistung ab. Andererseits ergibt sich an der Schneidelektrode 48 eine Stromkonzentration, sodass nun trotz geringerer Leistung ein Schnitt durchgeführt werden kann.

Wie die schematische Darstellung nach Figur 7 zeigt, kann die Aktivierung der verschiedenen Schaltstrecken 21, 22, 23 zu einer Verschiebung der Resonanzfrequenz f des reaktiven Netzwerks 19 bzw. transformierten reaktiven Netzwerks 19* führen. Alle drei den Schaltstrecken 21, 22, 23 zugeordneten Resonanzfrequenzen f21, f22, f23 können unterhalb der Frequenz f43 des Schaltsignals 43 liegen. Es ist prinzipiell aber auch möglich, eine oder mehrere der Frequenzen F21, F22, F23 oberhalb des Schaltsignals 43 festzulegen.

Bei allen vorstehend beschriebenen Ausführungsformen wurde vorausgesetzt, dass der Schalter 40 mit einer fest vorgegebenen Frequenz geöffnet und geschlossen wird, um den Schwingkreis 24 zu schwingen anzuregen. Es ist aber bei allen Ausführungsformen auch möglich, den Schwingkreis bei seiner Eigenresonanz schwingen zu lassen, indem das Steuersignal 43 für den Schalter 40 aus der Schwingfrequenz des Schwingkreises 24 abgeleitet wird. Der Schwingkreis 24 ist dann das frequenzbestimmende Element der so gebildeten Oszillatorschaltung.

Mit dem erfindungsgemäßen Konzept wird ein Generator 11 zur Speisung verschiedener Instrumente 35, 44 vorgeschlagen, der ohne Zuhilfenahme einer Regelschleife gewünschte Ausgangskennlinien bereitstellt. Dazu nutzt er ein reaktives Netzwerk 19, dass die gewünschten Kennlinien von sich aus (d.h. intrinsisch) hat. Dies wird erreicht, indem in dem Ausgangszweig des Generators 11 verschieden wahlweise einschaltbare komplexe Widerstände vorgesehen sind, die unterschiedliche Zusammenhänge zwischen abgegebener Leistung und Lastwiederstand bereitstellen.

### Bezugszeichen:

- 10: Lastwiderstand
- 11: Generator
- Z_{L}: Last-Impedanz
- R: Betrag der Last-Impedanz Z_{L}
- 12, 13: Leitungen
- 14, 15: Pole
- 16: Generatorausgang
- 17: hochfrequente Spannungsquelle
- 18: komplexer Innenwiderstand
- Zᵢ: Impedanz des Innenwiderstands 18
- Z₁, Z₂, Z₃: Impedanzen des Innenwiderstands 18
- 19: reaktives Netzwerk
- 19*: transformiertes reaktives Netzwerk
- 20: Wahlschalter
- 21 - 23: Schaltstrecken des Wahlschalters
- S: Steuersignal
- 24: Schwingkreis
- 25: Primärkondensator
- 26: Primärinduktor
- 27: Hochfrequenztransformator
- 28, 30: Sekundärinduktoren
- 31: Koppelkondensator
- 32, 34: Sekundärkondensatoren
- 35: Instrument
- 36: Elektrode
- 37: biologisches Gewebe
- 38: Neutralelektrode
- 39: Schalter
- 40: Steuerstrecke
- 41: Steuereingang
- 42: Taktgeber
- 43: Steuersignal
- 44: Instrument
- 45, 46: Branchen
- 45a, 45b: Teilelektroden der Branche 45
- 46a, 46b: Teilelektroden der Branche 46
- 47: Gewebe
- 48: Schneidelektrode
- 49: Widerlager
- Z*_{L}: transformierte Last-Impedanz
- L*_{S}: transformierte Sekundärinduktivität
- C*_{S}: transformierte Sekundärkapazität

## Patentansprüche

1. Generator (10) zur Speisung eines elektrochirurgischen Instruments (35, 44) mit einem eine Gewebeveränderung, insbesondere eine devitalisierende Gewebeveränderung hervorrufen Strom,
wobei der Generator (11) einen Resonanzkreis (24) mit einer Resonanzfrequenz (f_{R}) umfasst, der einen primärseitigen Induktor (26) und einen dazu parallel geschalteten primärseitigen Kondensator (25) aufweist,
mit mindestens einem elektronischen Schalter (39), der eine an den Resonanzkreis (24) angeschlossene Steuerstrecke (40) und einen Steuereingang (41) aufweist, über den die Steuerstrecke (40) alternierend zwischen einem leitenden und einem nichtleitenden Zustand umschaltbar ist,
mit einem Taktgeber (42), der zur Erzeugung eines Schaltsignals (43) eingerichtet und mit dem Steuereingang (41) verbunden ist, um die Steuerstrecke (40) ein und aus zu schalten,
mit einem ersten und einem zweiten sekundärseitigen Induktor (28, 29), die mit dem primärseitigen Induktor (26) induktiv gekoppelt sind,
mit einem ersten sekundärseitigen Kondensator (32), der mit dem ersten sekundärseitigen Induktor (28) in Reihe geschaltet ist und mit einem zweiten sekundärseitigen Kondensator (33), der mit dem zweiten sekundärseitigen Induktor (29) in Reihe geschaltet ist, sowie
mit einem Wahlschalter (20), der zwei Schaltstrecken (21, 22) aufweist, von denen eine mit dem ersten sekundärseitigen Induktor (28) und dem ersten sekundärseitigen Kondensator (32) und die andere mit dem zweiten sekundärseitigen Induktor (29) und dem zweiten sekundärseitigen Kondensator (33) in Reihe geschaltet ist.

2. Der erste sekundärseitige Induktor (28) und der erste sekundärseitige Kondensator (32) miteinander in Reihe geschaltet sind.

3. Der Generator (11) weitere mit dem primärseitigen Induktor (28) induktiv gekoppelte sekundärseitige Induktoren (29, 30) aufweist, die jeweils mit weiteren sekundärseitigen Kondensatoren (34, 35) in Reihe geschaltet sind, wobei der Wahlschalter (20) dazu eingerichtet ist, zu jedem Zeitpunkt lediglich eine aus einem sekundärseitigen Induktor (28) und sekundärseitigem Kondensator (33) bestehende Reihenschaltung (28, 33) mit dem Generatorausgang (16) zu verbinden.

4. Bei jeder Einstellung des Wahlschalters (20) von den Schaltstrecken (21, 22, 23) jeweils nur eine ausgewählte Schaltstrecke (21 oder 22 oder 23) leitend ist und alle anderen nichtleitend sind.

5. Der Taktgeber (42) dazu eingerichtet ist, das Steuersignal (41) mit vorgegebener Frequenz zu generieren.

6. Der Taktgeber (42) dazu eingerichtet ist, das Steuersignal (41) mit unveränderlicher Frequenz zu generieren.

7. Der Generator (11) bei unterschiedlichen Einstellungen des Wahlschalters (20) unterschiedliche Innenwiderstände (Z₁, Z₂, Z₃) aufweist.

8. Die sekundärseitigen Kondensatoren (32, 33, 34) und sekundärseitigen Induktivitäten (28, 29, 30) so bemessen sind, dass die Resonanzfrequenz des Parallelschwingkreises größer als 200 kHz ist.

9. Der Taktgeber (42) darauf eingerichtet ist, das Schaltsignal (43) mit einer Schaltfrequenz (f) abzugeben, die größer als die Resonanzfrequenz (f_{R}) des Schwingkreises (24).

10. Alle sekundärseitigen Induktoren (28, 29, 30) miteinander in Reihe geschaltet sind.

11. Die sekundärseitigen Kondensatoren (32, 33, 34) unterschiedliche Kapazitätswerte aufweisen.

12. Die Kapazitätswerte der sekundärseitigen Kondensatoren (32, 33, 34) umso kleiner sind, je mehr sekundärseitige Induktoren (28, 29, 30) mit ihnen in Reihe geschaltet sind.
